# EUROPÄISCHE PATENTSCHRIFT

(11) **EP 0 341 446 B1**
(45) Veröffentlichungstag und Bekanntmachung des Hinweises auf die Patenterteilung: **14.09.1994**
(21) Anmeldenummer: 89106759.7
(22) Anmeldetag: 14.04.1989
(51) Int. Cl.: A61B 17/39

(54) **Hochfrequenzgenerator zum Gewebeschneiden und Koagulieren in der Hochfrequenzchirurgie**
High frequency generator to cut and coagulate the tissues in high frequency surgery
Générateur à haute fréquence pour découper et coaguler les tissus en chirurgie haute fréquence

(30) Priorität: 09.05.1988 DE 3815835
(43) Veröffentlichungstag der Anmeldung: 15.11.1989
(73) Patentinhaber: Karl Storz GmbH & Co., D-78532 Tuttlingen (DE)
(72) Erfinder: Flachenecker, Gerhard, Prof.-Dr.-Ing., 8012 Ottobrunn (DE); Fastenmeier, Karl, Prof.-Dr.-Ing., D-8000 München 83 (DE); Lindenmeier, Heinz, Prof.-Dr.-Ing., D-8033 Planegg (DE)
(74) Vertreter: Münich, Wilhelm, Dr.

(56) Entgegenhaltungen:
- EP-A- 0 237 795
- DE-C- 2 504 280
- FR-A- 2 502 935
- US-A- 4 092 986

## Beschreibung

Die Erfindung bezieht sich auf einen Hochfrequenzgenerator zum Gewebeschneiden und Koagulieren in der Hochfrequenzchirurgie entsprechend dem Oberbegriff des Anspruchs 1.

In der Hochfrequenzchirurgie werden Hochfrequenzströme zum Schneiden und Koagulieren menschlichen Gewebes verwendet. Beim Schneiden wird ein annähernd kontinuierlicher Hochfrequenzstrom zugeführt. Dabei bildet sich zwischen der Hochfrequenzchirurgiesonde und dem zu schneidenden Gewebe ein kleiner Lichtbogen aus. Der Hochfrequenzgenerator muß eine Spannung abgeben, die zum Zünden des Lichtbogens ausreicht. Der in das Gewebe übertretende Hochfrequenzstrom erwärmt das an der Hochfrequenzchirurgiesonde anliegende Gewebe so stark, daß die Zellflüssigkeit explosionsartig verdampft und das Gewebe dabei trennt.

Beim Koagulieren wird die Hochfrequenzleistung impulsförmig zugeführt. Dabei ist die mittlere zugeführte Leistung klein genug, daß es nicht mehr zur explosionsartigen Dampfentwicklung kommt. Die Zellflüssigkeit entweicht vielmehr langsam und das Gewebe trocknet aus, ohne getrennt zu werden. Dabei wird das Gewebe allerdings hochohmig. Soll eine tiefe Koagulationswirkung erzielt werden, so muß die Hochfrequenzspannung relativ hohe Impulsamplituden haben, um den Stromfluß durch die hochohmige Oberfläche hindurch aufrecht zu erhalten.

Beim Gewebeschneiden wird dem Patienten eine relativ hohe mittlere Leistung zugeführt. Dies birgt für den Patienten die bekannten Gefahren, wie z.B. Verbrennungen. Es ist daher sehr wichtig, die Leistung des Hochfrequenzgenerators so klein wie möglich einzustellen. Die Ausgangsspannung muß so gewählt werden, daß gerade ein genügender Gewebe-Trenneffekt auftritt, aber keine überschüssige Leistung an den Patienten abgegeben wird.

Es ist bekannt, daß eine manuelle Generatoreinstellung beim Schneiden immer zu unbefriedigenden Ergebnissen führt. Aus diesem Grund wird in der Deutschen Patentschrift 25 04 280 eine Vorrichtung zum elektrischen Gewebeschneiden in der Chirurgie beschrieben, bei der der momentane Zustand des Schneidvorganges mithilfe elektrischer Signale überwacht wird. Mit einer Regeleinrichtung wird die Ausgangsspannung so eingestellt, daß der Schneidvorgang auf einen gewünschten Zustand konstant geregelt wird. In einer Ausgestaltung wird dazu die Intensität des Lichtbogens, der zwischen der Chirurgiesonde und dem Gewebe brennt, gemessen und konstant geregelt. Diese Lichtbogen-Regelung bringt eine deutliche Reduzierung der mittleren, dem Patienten zugeführten Leistung gegenüber manuell einstellbaren Hochfrequenzgeneratoren.

Bei der beschriebenen Regelung kann die Leerlaufspannung des Hochfrequenzgenerators für das Gewebeschneiden auf jeden beliebigen Wert festgelegt werden, der über der maximalen, von der Regelung je eingestellten Spannung liegt. In der Praxis wählt man daher die Leerlaufspannung des Hochfrequenzgenerators so, daß sie dem gewünschten Wert für die Impulsspitzen beim Koagulieren entspricht. Auf diese Weise kann man einen sehr einfachen und kostengünstigen Hochfrequenzgenerator bauen, bei dem die Hochfrequenzleistungen für Schneiden und Koagulieren mit dem gleichen Leistungsgenerator erzeugt werden.

Mit umfangreichen Messungen haben die Erfinder jedoch herausgefunden, daß diese Maßnahme in der Praxis zu gewissen Nachteilen führt. In der Urologie zum Beispiel werden die Gewebeschnitte bei Anwesenheit von Spülflüssigkeit durchgeführt. Als Spülflüssigkeit werden zwar bevorzugt ionenarme und damit hochohmige Wässer verwendet. Nach den ersten Schnitten ist der Spülflüssigkeit aber immer etwas Blut beigemengt. Damit ergibt sich eine erhöhte Leitfähigkeit der Spülflüssigkeit. Wird der Hochfrequenzgenerator versehentlich oder absichtlich aktiviert, solange kein Kontakt zwischen der Chirurgiesonde und dem Gewebe besteht, so kann kein Lichtbogen entstehen und die Regelung stellt die maximal mögliche Hochfrequenz-Ausgangsspannung ein. Dies ist annähernd die Leerlaufspannung des Hochfrequenzgenerators. Dem Patienten fließt dabei über die Spülflüssigkeit ein umso höherer Strom zu, je höher die Leerlaufspannung des Hochfrequenzgenerators gewählt ist. In Wirklichkeit sollte dieser Strom jedoch so klein wie möglich sein, um den Patienten nicht unnötig zu belasten.

In der offenen Chirurgie, also ohne Anwesenheit von Spülflüssigkeit, kann der Operateur den Hochfrequenzgenerator z.B. aktivieren, bevor er das Gewebe mit der Chirurgiesonde berührt. Solange kein Kontakt mit dem Gewebe existiert, kann auch kein Lichtbogen zünden. Auch in diesem Fall wird die Ausgangsspannung des Hochfrequenzgenerators praktisch auf die Leerlaufspannung hochgeregelt. Wegen der endlichen Regelgeschwindigkeit der Lichtbogen-Regelung entsteht bei der ersten Berührung mit dem Gewebe zunächst ein stärkerer Lichtbogen als mit der Lichtbogen-Regelung vorgewählt wurde. Dieser Initial-Lichtbogen ist umso stärker, je höher die Leerlaufspannung des Hochfrequenzgenerators ist. Er erzeugt an der Anschnittstelle eine stärkere Nekrose als erwünscht ist.

Der Erfindung liegt daher die Aufgabe zugrunde, einen Hochfrequenzgenerator mit Lichtbogen-Regelung derart zu verbessern, daß die geschilderten Nachteile vermieden werden, d.h. die Ausgangsspannung und die Ausgangsleistung so klein wie möglich gehalten werden, solange kein Lichtbogen zwischen der Chirurgiesonde und dem Gewebe brennt.

Diese Aufgabe wird mit den Maßnahmen gelöst, die in den Kennzeichen des Anspruchs 1 und der Unteransprüche vorgeschlagenen werden.

Der Hochfrequenzgenerator nach der Erfindung enthält eine Meßeinrichtung, die die Ausgangsspannung des Hochfrequenzgenerators mißt. Das Meßergebnis wird einer Spannungs-Begrenzerschaltung zugeführt, die auf den Leistungsgenerator zurückwirkt und die Ausgangsspannung des Hochfrequenzgenerators im Betriebsmodus "Schneiden" auf einen vorgegebenen Maximalwert begrenzt, damit die Ausgangsspannung aufgrund der Lichtbogenregelung nicht über diesen Maximalwert ansteigen kann. Dieser Maximalwert kann so gewählt werden, daß er in allen praktischen Fällen, in denen die Chirurgiesonde Gewebe schneidet, durch die Lichtbogen-Regelung nicht überschritten wird. Damit ist die Spannungsbegrenzung nur dann wirksam, wenn die Chirurgiesonde das Gewebe nicht berührt. Auf diese Weise kann die dem Patienten zugeführte Hochfrequenzleistung wirksam reduziert werden, ohne die Qualität des Schneidvorganges zu beeinträchtigen.

In einer Ausgestaltung der Erfindung wird die Hochfrequenz-Ausgangsspannung von der Meßeinrichtung direkt am Ausgang des Hochfrequenzgenerators gemessen. Die Spannungsbegrenzung ist damit am wirksamsten.

Die Ausgangsspannung des Hochfrequenzgenerators hat allerdings hohe Amplituden und eine direkte Messung ist u.U. schwierig zu realisieren. In einer weiteren Ausgestaltung der Erfindung ist daher vorgesehen, daß die Meßeinrichtung die Ausgangsspannung im Ausgangsfilter mißt. Im Ausgangsfilter können Punkte gefunden werden, an denen Bruchteile der Ausgangsspannung anliegen, die entweder proportional oder wenigstens ungefähr proportional zur Ausgangsspannung sind. Diese Spannungen sind von der Meßeinrichtung leichter zu verarbeiten und können ebenfalls zur Spannungsbegrenzung verwendet werden.

In vielen Fällen werden im Hochfrequenzgenerator Leistungsgeneratoren verwendet, die im Schaltbetrieb arbeiten. Solche Leistungsgeneratoren haben einen sehr hohen Wirkungsgrad und werden deshalb in der Hochfrequenzchirurgie häufig verwendet. Die Leistungsregelung solcher Generatoren geschieht über eine Regelung der Betriebsspannung. Dabei ist die Amplitude der Hochfrequenz-Ausgangsspannung nahezu streng proportional zur Betriebsspannung. In einer Ausgestaltung der Erfindung wird daher ein Leistungsgenerator verwendet, der im Schaltbetrieb arbeitet, wobei die Meßeinrichtung die Betriebsspannung des Leistungsgenerators mißt und daraus das Signal für die Spannungsbegrenzung ableitet.

Umfangreiche Messungen der Erfinder haben ergeben, daß die maximalen Hochfrequenz-Ausgangsspannungen, die von einer Lichtbogenregelung eingestellt werden, sehr vom Anwendungsfall abhängen. In der Urologie, bei Unterwasserschnitten, liegen die maximalen Ausgangsspannungen bei langsamen Schnitten an Blasentumoren etwa bei 250 bis 300 V. Bei schnellen, tiefen Schnitten an Prostata-Tumoren oder Prostata-Karzinomen werden dagegen Werte von 350 bis 450 V erreicht. Eine Ausgestaltung der Erfindung sieht daher bei Hochfrequenzgeneratoren für Anwendungen mit Spülflüssigkeiten einen Maximalwert der Hochfrequenz-Ausgangsspannung von 250 bis 450 V vor.

In der offenen Chirurgie, ohne Anwesenheit von Spülflüssigkeiten, wurden deutlich geringere maximale Ausgangsspannungen gemessen. Bei Anwendung von Nadelelektroden liegen die maximalen von der Lichtbogenregelung eingestellten Hochfrequenz-Ausgangsspannungen bei etwa 100 V, bei Verwendung von Hochfrequenz-Skalpellen bei etwa 250 V. Eine Ausgestaltung der Erfindung sieht daher bei Hochfrequenzgeneratoren für Anwendungen ohne Spülflüssigkeiten einen Maximalwert der Hochfrequenz-Ausgangsspannung von 100 bis 250 V vor.

Zur weiteren Verdeutlichung der Erfindung sind noch Bilder beigefügt. Es zeigen:
- Fig. 1:: Hochfrequenzgenerator mit im Schaltbetrieb arbeitendem Leistungsgenerator und mit Messung der Ausgangsspannung im Ausgangsfilter.
- Fig. 2:: Hochfrequenzgenerator mit im Schaltbetrieb arbeitendem Leistungsgenerator und mit Messung der Betriebssspannung des Leistungsgenerators.
- Fig. 3:: Hochfrequenzgenerator mit linear arbeitendem Leistungsgenerator und mit Messung der Ausgangsspannung im Ausgangsfilter.
- Fig. 4:: Verbundwahrscheinlichkeit für Impedanz und Leistung bei einer Prostata-Resektion mit einem Hochfrequenzgenerator ohne Spannungsbegrenzung.
- Fig. 5:: Verbundwahrscheinlichkeit für Impedanz und Leistung bei einer Prostata-Resektion mit einem Hochfrequenzgenerator mit Spannungsbegrenzung.

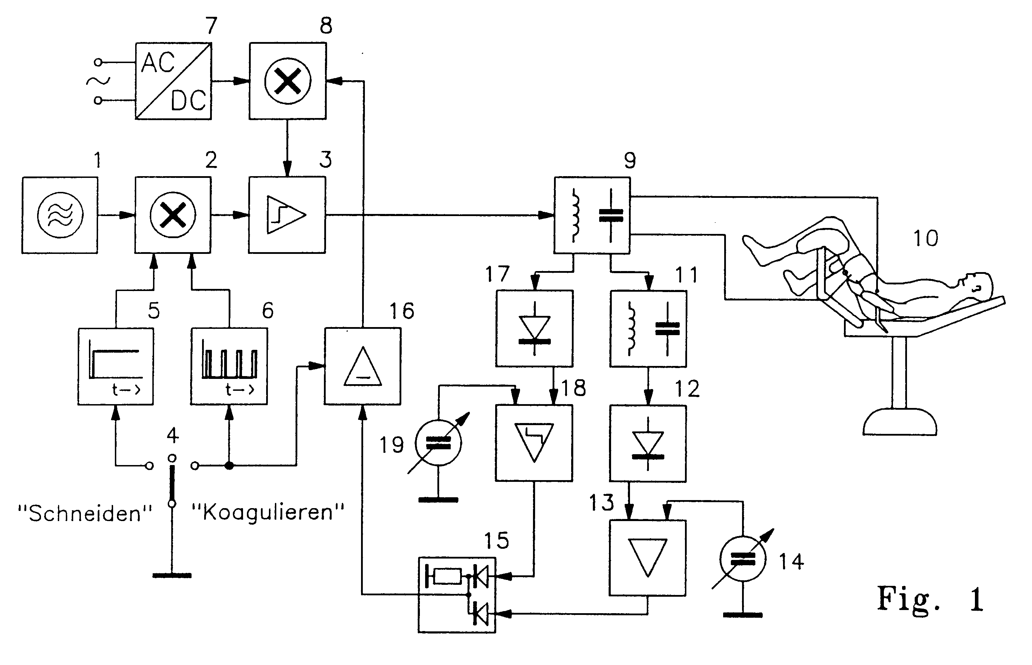

In Fig. 1 ist als Beispiel das Blockschaltbild eines Hochfrequenzgenerators nach der Erfindung mit im Schaltbetrieb arbeitendem Leistungsgenerator und mit Messung der Ausgangsspannung im Ausgangsfilter dargestellt. Der Oszillator 1 steuert über den Modulator 2 den Leistungsverstärker 3. Mit dem Modulator 2 kann das Zeitverhalten des Hochfrequenzgenerators gesteuert werden. Dazu wird der Modulator 2 vom Schalter 4, der in den Stellungen "Schneiden" oder "Koagulieren" aktiviert werden kann, über die Schaltglieder 5 und 6 angesteuert. Diese Zeitglieder aktivieren den Modulator entweder ständig, wie beim Schneiden, oder impulsförmig, wie beim Koagulieren.

Diese Art der Modulation ist nur ein einfaches Beispiel aus vielen Möglichkeiten. Es sind z.B. Hochfrequenzgeneratoren bekannt, bei denen das Schaltglied 5 auch eine zeitliche Begrenzung des Aktivierungszustandes durchführt. Insbesondere für das Schaltglied 6 sind noch wesentlich kompliziertere Lösungen mit zusätzlichen Funktionen, wie z.B. lichtbogenabhängige Umschaltung zwischen Dauerleistung und Impulsleistung bekannt geworden. Für die Erläuterung der Erfindung reichen die einfachen Schaltglieder 5 und 6 der Fig. 1 jedoch aus.

Der Leistungsgenerator 3 arbeitet im Schaltbetrieb. Er wird über den Netzgleichrichter 7 und den Gleichspannungsregler 8 mit Betriebsspannung versorgt. Mit dem Gleichspannungsregler 8 kann die Hochfrequenz-Ausgangsspannung des Leistungsgenerators eingestellt werden. Diese Ausgangsspannung wird über das Ausgangsfilter 9 in bekannter Weise zur Chirurgiesonde, d.h. an den Patienten 10 weitergeleitet.

Aus dem Ausgangsfilter wird ein Signal über den Zustand des Schneidvorganges abgezweigt. Im vorliegenden Beispiel wird hierfür das Filter 11 verwendet. Es ist auf eine oder mehrere zur Generatorfrequenz harmonische Frequenzen abgestimmt. Diese Frequenzen entstehen durch das nichtlineare Verhalten des an der Chirurgiesonde brennenden Lichtbogens. Ihre Amplituden sind ein direktes Maß für die Intensität des Lichtbogens und können zu einer Regelung der Lichtbogenintensität verwendet werden. Dazu werden die im Filter 11 ausgefilterten Harmonischen im Gleichrichter 12 gleichgerichtet und im Differenzverstärker 13 mit einem einstellbaren Sollwert aus dem Sollwertgeber 14 verglichen. Das Ergebnis dieses Vergleichs gelangt über das Additionsglied 15 und den Regelverstärker 16 auf den Gleichspannungsregler 8. Damit ist der Regelkreis geschlossen und die Lichtbogenintensität wird automatisch auf den vom Sollwertgeber 14 vorgeschriebenen Wert geregelt.

Da diese Regelung nur im Betriebsmodus Schneiden wirksam sein soll, wird der Regelverstärker 16 in Stellung "Koagulieren" des Schalters 4 deaktiviert. Um die richtige Phase für den Regelkreis zu gewährleisten, ist der Regelverstärker 16 als Umkehrverstärker ausgeführt.

Aus dem Ausgangsfilter 9 wird nun weiterhin eine der Ausgangsspannung des Hochfrequenzgenerators proportionale Spannung abgegriffen und dem Gleichrichter 17 zugeführt. Dessen Ausgangssignal wird im Komparator 18 mit einem vom Sollwertgeber 19 gelieferten Sollwert verglichen. Wenn das vom Gleichrichter 17 gelieferte Signal den vom Sollwertgeber 19 gelieferten Wert übersteigt, greift das Ausgangssignal des Komparators 18 über das Additionsglied 15 in den Regelvorgang ein und verhindert ein Ansteigen der Hochfrequenz-Ausgangsspannung über den vom Sollwertgeber 19 vorgeschriebenen Wert. Auf diese Weise ist die Hochfrequenz-Ausgangsspannung auf den vorgegebenen Wert begrenzt.

Der Wert des Sollwertgebers 19 muß natürlich die Konversionsfaktoren des Ausgangsfilters 9 und des Gleichrichters 17 berücksichtigen.

Es muß erwähnt werden, daß in diesem Beispiel aus Gründen der Anschaulichkeit eine sehr einfache Lichtbogen-Regelung und eine sehr einfache Spannungsbegrenzung gezeigt wird. In der Praxis können zur Erzielung besserer Regel- und Begrenzereigenschaften wesentlich umfangreichere Schaltungen nach an sich bekannten regelungstechnischen Grundsätzen realisiert werden.

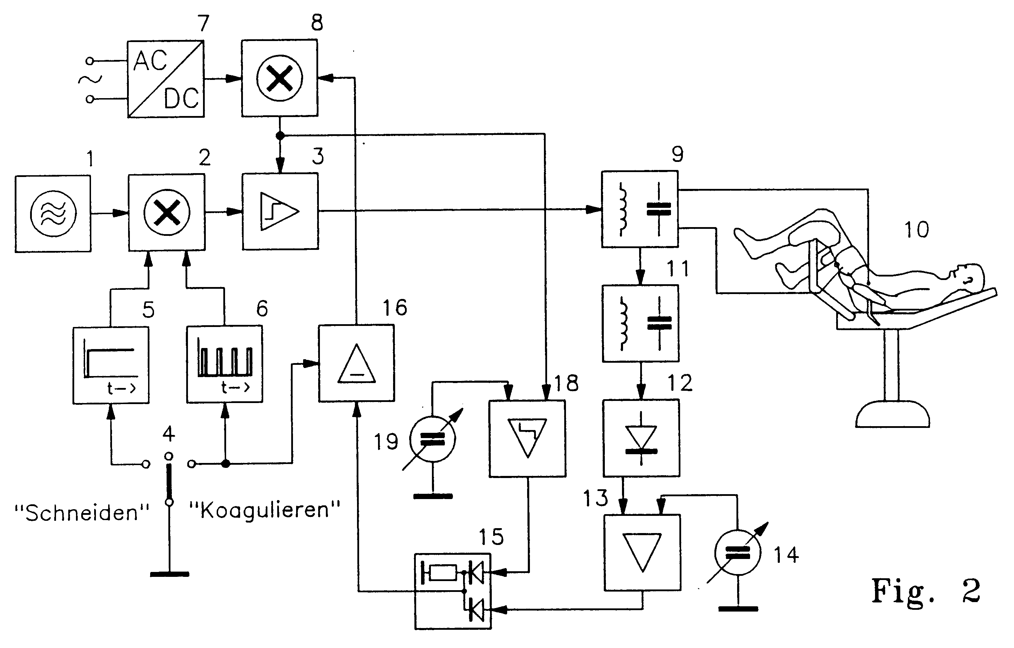

Fig. 2 zeigt das Blockschaltbild einer ähnlichen Schaltung wie in Fig. 1. Da in diesen Beispielen ein Leistungsgenerator mit Schaltbetrieb verwendet wird, kann die Betriebsspannung des Leistungsgenerators als Maß für seine Hochfrequenz-Ausgangsspannung verwendet werden. Dem Komparator 18 wird daher die Ausgangsspannung des Gleichspannungsreglers 8 als Signal zugeführt und mit dem Wert des Sollwertgebers 19 verglichen. Natürlich muß auch hier beim Wert des Sollwertgebers 19 der Umrechnungsfaktor zwischen der Betriebsspannung des Leistungsgenerators 3 und dessen Hochfrequenz-Ausgangsspannung berücksichtigt sein.

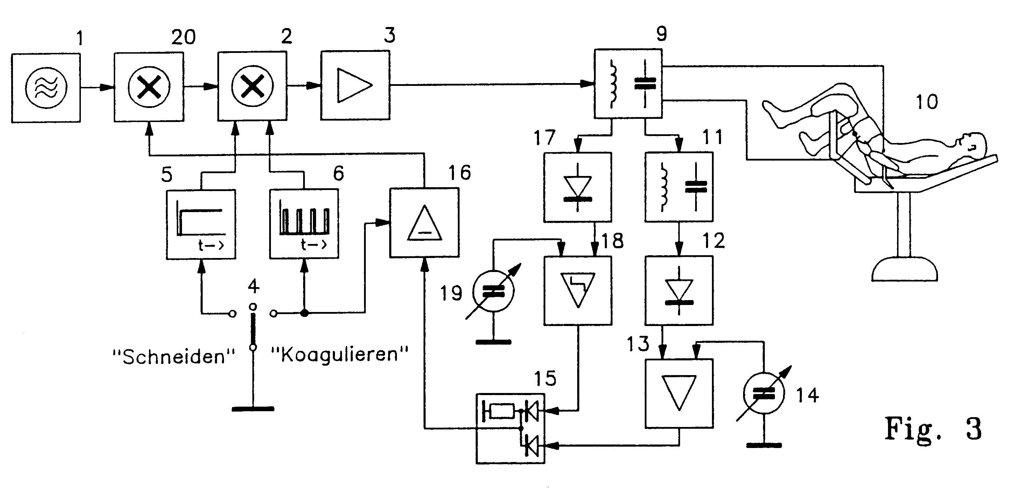

In Fig. 3 ist das Blockschaltbild eines Hochfrequenzgenerators mit linearem Leistungsgenerator dargestellt. Da hier keine Regelung der Hochfrequenz-Ausgangsspannung über die Betriebsspannung des Leistungsgenerators erfolgen kann, ist die Betriebsspannungs-Versorgung in diesem Beispiel gar nicht gezeichnet. Dafür ist hier ein weiterer Modulator 20 vorgesehen, der vom Ausgangssignal des Regelverstärkers 16 gesteuert wird und die entsprechende Lichtbogen-Regelung und Spannungsbegrenzung durchführt.

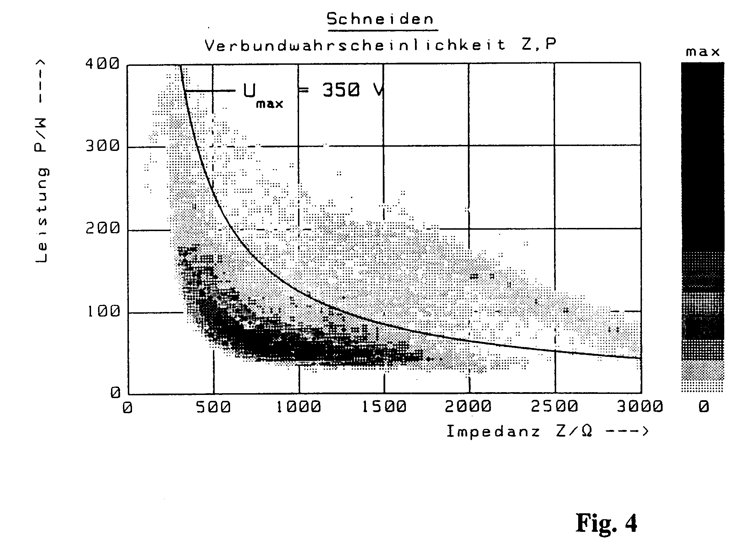

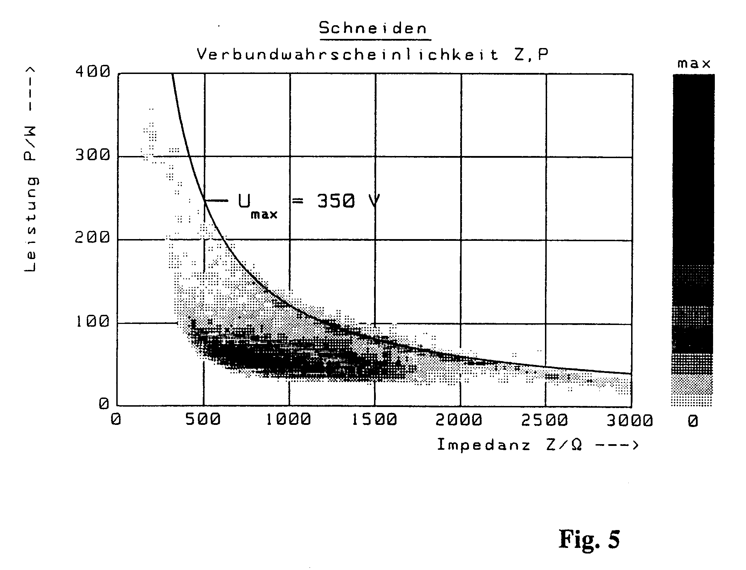

In den Figuren 4 und 5 ist gezeigt, welche Fortschritte mit einem Hochfrequenzgenerator nach der Erfindung erzielt werden können. In Fig. 4 ist das Ergebnis einer meßtechnisch vollständig aufgezeichneten Prostata-Resektion aufgetragen. Als Darstellungsform wurde die Verbundwahrscheinlichkeit für Impedanz und Leistung beim Schneiden gewählt. Bei der Operation wurden etwa 50 g Tumorgewebe entfernt. Die Messung wurde mit einer Abtastrate von ca. 50 Messungen/Sekunde durchgeführt. Dabei wurden etwa 30.000 Meßwerttripel für Spannung, Strom und Phasenverschiebung aufgezeichnet, aus denen nachträglich die Werte für Leistung und Impedanz berechnet wurden. In Fig. 4 sind die Meßwertkombinationen aus Impedanz und Leistung entsprechend ihrer Häufigkeit, d.h. ihrer Wahrscheinlichkeit in verschiedenen Schwärzungsgraden eingetragen. Am rechten Rand ist zur Eichung eine entsprechende Grauskala angegeben.

In der Verbundwahrscheinlichkeit der Fig. 4 ist links unten eine deutliche Häufung zu erkennen. Ausführliche Analysen der Meßergebnisse haben gezeigt, daß es sich dabei um die normalen Schneidvorgänge bei arbeitender Lichtbogen-Regelung handelt. Der hochsteigende Ast bei etwa 400 Ohm und 200 bis 400 W stellt den erhöhten Leistungsbedarf beim Anschneiden dar, wobei auch hier die Lichtbogen-Regelung normal arbeitet.

In Fig. 4 ist weiterhin eine mit Uₘₐₓ = 350 V bezeichnete Kurve eingetragen. Sie stellt den geometrischen Ort aller Meßwerte dar, bei denen die momentane Hochfrequenz-Ausgangsspannung des Hochfrequenzgenerators gerade 350 V war. Die von den Erfindern durchgeführten weitergehenden Analysen der Meßergebnisse haben ergeben, daß die Kurve bei dieser Operation etwa die Grenze zwischen normalen Schneidvorgängen und der Leistungsabgabe an die Spülflüssigkeit liegt: Alle Meßwerte, die oberhalb dieser Kurve liegen, erhöhen die Leistungsabgabe an den Patienten wie oben beschrieben, ohne für den Schneidvorgang relevant zu sein. Wäre bei dieser Operation die Ausgangsspannung des Hochfrequenzgenerators auf 350 V begrenzt gewesen, so hätten alle jenseits der eingezeichneten Kurve gemessenen Werte in ihrer Leistung deutlich reduziert werden können, ohne daß der Operateur einen Verlust an Schneidqualität festgestellt hätte.

In Fig. 5 sind schließlich noch die Meßwerte einer zu Fig. 4 vergleichbaren Prostata-Resektion aufgetragen, bei der der Hochfrequenzgenerator jedoch nach der Erfindung gestaltet war. Die Verbundwahrscheinlichkeit für Impedanz und Leistung zeigt wegen der Begrenzung der maximalen Ausgangsspannung auf 350 V einen deutlichen Rückgang der mittleren Leistung.

## Patentansprüche

1. Hochfrequenzgenerator zum Gewebeschneiden und Koagulieren in der Hochfrequenzchirurgie, bestehend aus einem Leistungsgenerator, der im Betriebsmodus Gewebeschneiden auf kontinuierliche Leistungsabgabe und im Betriebsmodus Koagulieren auf gepulste Leistungsabgabe geschaltet werden kann, und eine Lichtbogen-Regelung, die beim Gewebeschneiden die Intensität des zwischen der Chirurgiesonde und dem Gewebe brennenden Lichtbogens auf einen vorgewählten Wert annähernd konstant regelt
dadurch gekennzeichnet, daß
eine Meßeinrichtung (17) vorhanden ist, die die Hochfrequenz-Ausgangsspannung des Hochfrequenzgenerators oder eine dazu proportionale Größe mißt und daraus ein Meßsignal erzeugt mit dessen Hilfe die Ausgangsspannung des Hochfrequenzgenerators im Betriebsmodus "Schneiden" über eine Spannungs-Begrenzerschaltung (15, 16, 18 und 19) auf einen vorgegebenen Maximalwert begrenzt wird, solange die Hochfrequenz-Ausgangsspannung aufgrund der Lichtbogen-Regelung (11, 12, 13, 14, 15 und 16) über diesen Maximalwert ansteigen würde.

2. Hochfrequenzgenerator nach Anspruch 1,
dadurch gekennzeichnet, daß
die Meßeinrichtung (17) die Hochfrequenz-Ausgangsspannung am Ausgang des Hochfrequenzgenerators mißt.

3. Hochfrequenzgenerator nach Anspruch 1,
dadurch gekennzeichnet, daß
die Meßeinrichtung (17) die Hochfrequenz-Ausgangsspannung im Ausgangsfilter (9) des Hochfrequenzgenerators mißt.

4. Hochfrequenzgenerator nach Anspruch 1,
dadurch gekennzeichnet, daß
der Leistungsgenerator (3) im Schaltbetrieb arbeitet und die Meßeinrichtung (17) die Betriebsspannung des Leistungsgenerators mißt.

5. Hochfrequenzgenerator nach dem Ansprüchen 1 bis 4 für Anwendungen bei Anwesenheit von Spülflüssigkeiten,
dadurch gekennzeichnet, daß
der vorgegebene Maximalwert der Hochfrequenz-Ausgangsspannung zwischen 250 und 450 Volt liegt.

6. Hochfrequenzgenerator nach den Ansprüchen 1 bis 4 für Anwendungen ohne Anwesenheit von Spülflüssigkeiten,
dadurch gekennzeichnet, daß
der vorgegebene Maximalwert der Hochfrequenz-Ausgangsspannung zwischen 100 und 300 Volt liegt.

## Claims

1. High-frequency generator for incisions in the tissue et for coagulation in high-frequency surgery, comprising a power generator adapted to be switched to continuous power output, in the tissue incision mode, and a pulsed power output, in the coagulation mode, we as well as an electric-arc regulator which, in the tissue incision mode, controls the intensity of the electric arc burning between the surgical probe and the tissue, so as to maintain it approximately constant at a selected value,
**characterized** in that
a measuring means (17) is provided which measures the high-frequency output voltage of the high-frequency generator or a parameter proportional thereto, and derives therefrom a measuring signal which is used, in the "incision" mode, to limit the output voltage of the high-frequency generator to a maximum level predetermined by means of a voltage-limiting circuit (15, 16, 18 et 19) as long as said high-frequency output voltage would rise beyond said maximum value as a result of the closed-loop control of said electric arc (11, 12, 13, 14, 15 et 16).

2. High-frequency generator according to Claim 1,
**characterized** in that
said measuring means (17) measures said high-frequency output voltage at the output of the high-frequency generator.

3. High-frequency generator according to Claim 1,
**characterized** in that
said measuring means (17) measures said high-frequency output voltage in the output filter (9) of the high-frequency generator.

4. High-frequency generator according to Claim 1,
**characterized** in that
said power generator (3) operates in the switching mode, and in that said measuring means (17) measures the operating voltage of said power generator.

5. High-frequency generator according to Claims 1 to 4, for applications involving the presence of rinsing fluids,
**characterized** in that
said predetermined maximum value of the high-frequency output voltage ranges between 250 and 450 Volts.

6. High-frequency generator according to Claims 1 to 4, for applications involving the presence of rinsing fluids,
**characterized** in that
said predetermined maximum value of said high-frequency output voltage ranges between 100 and 300 Volts.

## Revendications

1. Oscillateur haute fréquence pour des coupes dans le tissu et la coagulation dans la chirurgie H.F., comprenant un oscillateur de puissance capable d'être commuté à une puissance utile continue, au mode de coupe dans le tissu, et à une puissance utile en pulsations, au mode de coagulation, et un régulateur d'arc électrique qui au mode de coupe dans le tissu, règle l'intensité de l'arc électrique établi entre la tête chirurgicale et le tissu de façon qu'il soit approximativement constant à un niveau choisi,
**caractérisé** en ce
qu'un moyen de mesure (17) est prévu qui mesure la tension de sortie H.F. de l'oscillateur haute fréquence ou un paramètre en proportionnel, et en dérive un signal de mesurage, au moyen duquel, au mode "de coupe", la tension de sortie de l'oscillateur haute fréquence est limitée à un niveau maximum déterminé moyennant un circuit limiteur de tension (15, 16, 18 et 19), pour un temps au cours duquel ladite tension de sortie H.F. s'augmenterait en dehors ledit niveau maximum en vertu de l'asservissement de l'arc électrique (11, 12, 13, 14, 15 et 16).

2. Oscillateur haute fréquence selon la Revendication 1,
**caractérisé** en ce
que les moyen de mesure (17) mesure ladite tension de sortie H.F. à la sorte de l'oscillateur haute fréquence.

3. Oscillateur haute fréquence selon la Revendication 1,
**caractérisé** en ce
que les moyen de mesure (17) mesure ladite tension de sortie H.F. dans le filtre de sortie (9) de l'oscillateur haute fréquence.

4. Oscillateur haute fréquence selon la Revendication 1,
**caractérisé** en ce
que les oscillateur de puissance (3) travaille au mode do commutation, et en ce que ledit moyen de mesure (17) mesure la tension de régime dudit oscillateur de puissance.

5. Oscillateur haute fréquence selon les Revendications 1 à 4 pour des applications à présence de fluides de lavage,
**caractérisé** en ce
que les niveau maximum de la tension de sortie H.F. se monte à une valeur entre 250 et 450 volt.

6. Oscillateur haute fréquence selon les Revendications 1 à 4 pour des applications à présence de fluides de lavage,
**caractérisé** en ce
que les niveau maximum définie de la tension de sorte H.F. se monte à une valeur entre 100 et 300 volt.
